# EUROPEAN PATENT APPLICATION

(11) **EP 3 400 933 A1**
(43) Date of publication of application: **14.11.2018**
(21) Application number: 18173387.4
(22) Date of filing: 26.11.2013
(51) Int. Cl.: A61K 9/14, A61K 31/519, A61P 35/00

(54) **NOVEL PHARMACEUTICAL TRAMETINIB COMPOSITION**

(30) Priority: 30.11.2012 US 201261731597 P
(62) Divisional of application: 13857873.7
(71) Applicant: Novartis AG, 4056 Basel (CH)
(72) Inventor: Campbell, Gossett, Collegeville, PA PA 19426 (US); Henriquez, Francisco, Collegeville, PA 19426 (US)
(74) Representative: Rudge, Sewkian

(57) **Abstract**

Disclosed are novel pharmaceutical formulations containing N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide solvate, methods of using the compositions in therapy and processes for preparing the same.

## Description

### FIELD OF THE INVENTION

The present invention relates to a powder for oral solution (POS) comprising N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide solvate, represented by the following formula (I), known as trametinib dimethyl sulfoxide, Mekinist® dimethyl sulfoxide, GSK1120212B and hereinafter also referred to as Compound A:

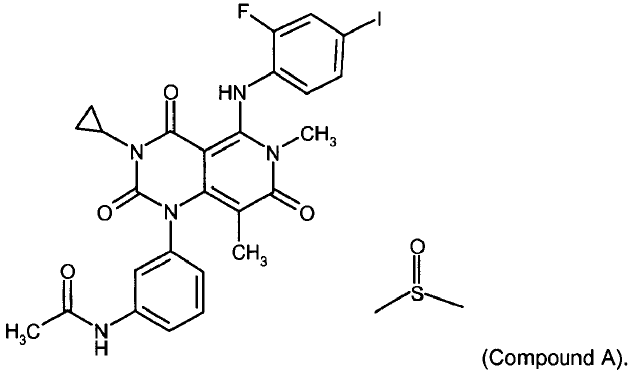

### BACKGROUND OF THE INVENTION

N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide, as the un-solvated compound (hereinafter Compound B) is a compound which is disclosed and claimed, along with pharmaceutically acceptable salts and solvates thereof, as being useful as an inhibitor of MEK activity, particularly in treatment of cancer, in International Application No. PCT/JP2005/011082, having an International filing date of June 10, 2005; International Publication Number WO 2005/121142 and an International Publication date of December 22, 2005, the entire disclosure of which is hereby incorporated by reference. Compound B is the compound of Example 4-1. Compound B can be prepared as described in International Application No. PCT/JP2005/011082. Compound B can be prepared as described in United States Patent Publication No. US 2006/0014768, Published January 19, 2006, the entire disclosure of which is hereby incorporated by reference. Compound B is the compound of Example 4-1.

Suitably, Compound B is in the form of a dimethyl sulfoxide solvate, or Compound A or trametinib dimethyl sulfoxide. Hereafter, "trametinib" means trametinib dimethyl sulfoxide. Suitably, Compound B is in the form of a solvate selected from: hydrate, acetic acid, ethanol, nitromethane, chlorobenzene, 1-pentanol, isopropyl alcohol, ethylene glycol and 3-methyl-1-butanol. Solvates and salt forms can be prepared by one of skill in the art, for example from the description in International Application No. PCT/JP2005/011082 or United States Patent Publication No. US 2006/0014768. Compound A is prepared in Example 4-149 of United States Patent Publication No. US 2006/0014768.

Solid oral pharmaceutical dosage forms are popular and useful forms of medications for dispensing pharmaceutically active compounds. A variety of such forms are known, including tablets, capsules, pellets, lozenges, and powders.

However, the formulation of an acceptable solid oral pharmaceutical dosage form on a commercial scale is not straightforward. When administered *in vivo,* each pharmaceutical compound acts uniquely in regards to therapeutic drug levels. Further, pharmaceutically active compounds, particularly anti-neoplastic compounds, are often associated with undesirable side effects such as; toxicity (e.g. genotoxicity, teratogenicity) and undesirable physical or psychological manifestations. In addition to balancing the drug's unique chemical properties with those of the excipients, the drug must be administered at a specific amount that is sufficient to provide the desired therapeutic drug level but less than the amount that presents an unacceptable side effect profile, or within the therapeutic window for that particular drug. Moreover, the formulation and process of manufacture must be such as to provide an integral dosage form that maintains its integrity until used. The dosage form must also possess acceptable dissolution and disintegration properties so as to provide the desired profile in use. Pharmaceutically active compounds with low solubility and/or in solvate form can present particular challenges in preparing high quality dosage forms. These challenges include insufficient and inconsistent exposure upon *in vivo* administration and desolvation which releases unsolvated compound that can exhibit poor pharmacodynamic properties.

Compound A is being evaluated in multiple tumor types and has shown anti-tumor activity in subjects with BRAF V600-mutation positive metastatic melanoma in a recent Phase III study. Compound A is currently being developed both as monotherapy and in combination with other anti-cancer medications including cytotoxic drugs and targeted small-molecule inhibitors. A New Drug Application (NDA) has been submitted to the FDA for approval of 0.5 mg, 1.0 mg and 2.0 mg tablets of Compound A. Solid dosage forms of Compound A, and specifically 0.5 mg, 1.0 mg and 2.0 mg tablets, are disclosed and claimed in International Application No. PCT/US2011/066021, having an International filing date of December 20, 2011; International Publication Number WO 2012/088033 and an International Publication date of June 28, 2012.

Though the disclosed tablets are acceptable for use in adults, the tablets are not preferred for administration of Compound A to children or individuals who have difficulty swallowing tablets. In pediatric populations, it is often desired that drug be available as a powder for reconstitution to an oral suspension or solution. Such a powder requires an attempt to dry blend various excipients with the active substance in the hope of providing a powder blend with good flow properties and content uniformity.

Several additional challenges exist concerning the use of Compound A in a pediatric formulation. For instance, the nature of the drug substance favors conversion from the dimethyl sulfoxide solvate to the de-solvated form in high humidity or an aqueous environment such that standard formulations fail to provide adequate physical stability and aqueous solubility. In addition, Compound A is very sensitive to light and therefore, packaging is a concern to the stability of the dosage form. Further, the drug has been found to have a bitter taste.

Significant realization of these concerns will have an adverse effect on the *in vivo* administration of Compound A.

It would be desirable to provide Compound A in a formulation suitable for administration to a pediatric population.

### SUMMARY OF THE INVENTION

The present invention is related to a powder for oral solution (POS) of Compound A which is adapted for reconstitution with water. This invention is also related to a prepared aqueous solution, formulation, particularly to a stable oral pharmaceutical formulation, comprising Compound A mixed with an aqueous vehicle. Additionally, the present invention is related to the method of preparing these formulations.

### BRIEF DESCRIPTION OF THE DRAWINGS

FIGURE 1: Figure 1 depicts the aqueous solubility of trametinib in the presence of various solubilizers.
FIGURE 2: Figure 2 depicts the concentration profile as a function of both time and Cavitron concentration from initial concentration of 1 mg/mL trametinib.
FIGURE 3: Figure 3 depicts the effect of HPMC and various solubilizers on the aqueous solubility of trametinib from initial concentration of 1 mg/mL trametinib.
FIGURE 4: Figure 4 depicts the solubility profile of Compound B at 0.05 mg/mL using various solubilizers as a function of time.
FIGURE 5: Figure 5 depicts the distance values between active and matching placebo of trametinib solutions with different flavoring systems and their discrimination indices

### DETAILED DESCRIPTION

In one embodiment, the present invention is directed to oral pharmaceutical dosage forms that contain Compound A, suitably the dosage forms are powder forms, suitably the dosage forms are produced on a commercial scale. These powder forms help provide safe and effective treatment.

In one embodiment, the present invention is directed to a prepared aqueous formulation, suitably to a stable oral aqueous pharmaceutical formulation, comprising Compound A mixed with excipients and aqueous vehicle. These prepared aqueous forms help provide safe and effective treatment.

As used herein, the term "powder for oral solution (POS)" means a pharmaceutical formulation containing pharmaceutical excipients and Compound A.

The direct powder blend of Compound A mixed with excipients is termed powder for oral solution (POS). Prior to administration, the POS is reconstituted with an aqueous vehicle to form a clear or slightly colored solution. The solution is dosed based on the body weight or body surface area of the patient.

It has been found that Compound A can suffer from photo-instability. The potential for unacceptable levels of photo-degradation is of particular importance since photo-catalyzed degradation products may be potentially toxic.

In one embodiment, the present invention is directed to powder for oral solution (POS) containing Compound A in an amount selected from: about 0.1% w/w, suitably less than 0.1% w/w, suitably about 0.043% w/w. These formulations help provide safe and effective treatment.

In one embodiment, the present invention is directed to powder for oral solution (POS) containing Compound A wherein the ratio of solubilizer to Compound A is greater than 100 to 1, suitably greater than 1000 to 1, suitably greater than 1500 to 1, suitably about 1771 to 1. These formulations help provide safe and effective treatment.

It has been found that Compound A can undergo desolvation during handling and formulation resulting in unsolvated Compound B being formed. Compound B is much less soluble than Compound A, which negatively impacts its pharmacodynamics when released from a pharmaceutical composition. Suitably the formulations of the present invention contain an amount of desolvated Compound B that does not exceed 30%, suitably does not exceed 25%, suitably does not exceed 20%, suitably does not exceed 15%, suitably does not exceed 10%, suitably does not exceed 5%, suitably does not exceed 2%, when compared to Compound A. Such formulations help provide safe and effective treatment.

It has been found that Compound A can exhibit poor exposure and absorption upon *in vivo* administration. Suitably the powder for oral solution (POS) of the current invention contain micronized Compound A where at least 90% of the particles of Compound A are from 1 to 20 microns, suitably from 2.2 to 10.5 microns, such formulations provide an acceptable exposure/absorption profile. Suitably the powder for oral solution (POS) of the current invention contains micronized Compound A where at least 50% of the particles of Compound A are from 1 to 6 microns, suitably from 1.5 to 4.3 microns, such formulations provide an acceptable exposure/absorption profile. Suitably the powder for oral solution (POS) of the current invention contains micronized Compound A where at least 10% of the particles of Compound A are from 0.01 to 3.0 microns, suitably from 0.77 to 1.3 microns, such formulations provide an acceptable exposure/absorption profile. Such formulations help provide safe and effective treatment.

In one embodiment, the particle size distribution of Compound A unmicronized particles in the currently invented powder for oral solution (POS) is that 90% of the particles of Compound A are not greater than 140 microns, suitably 120 microns or less. Such formulations provide an acceptable exposure/absorption profile. Such formulations help provide safe and effective treatment.

As used herein, the term "improved properties" and derivatives thereof, contemplates several advantages to the pharmacokinetic profile of the *in vivo* release of Compound A from a POS that utilizes an aspect of the present invention when compared to a formulation that does not utilize that aspect of the present invention, suitably the formulation is produced on a commercial scale. Examples of improved properties include: increased oral bioavailability, improved physical and chemical stability, improved photo-stability, a consistent pharmacokinetic profile, an improved pharmacokinetic profile, a consistent dissolution rate and a stable oral pharmaceutical formulation when the POS is mixed with an aqueous vehicle.

As used herein, the term "drug" or "active ingredient" and derivatives thereof, unless otherwise defined, means Compound A or N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide.

As used herein, the term "Compound B" and derivatives thereof, means N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide, as the free or unsalted and unsolvated compound. Compound B also refers to the amount of free or unsalted and unsolvated compound in an amount of Compound A.

By the term "commercial scale" and derivatives thereof, as used herein is meant, preparation of a batch scale greater than about 20 kg of POS, suitably greater than 50 kg, suitably greater than 75 kg or a batch size suitable to prepare at least about 10,000 POS doses, suitably at least 25,000 doses, suitably at least 50,000 doses.

The term "effective amount" and derivatives thereof, means that amount of a drug or active ingredient that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

By the term "co-administration" as used herein is meant either simultaneous administration or any manner of separate sequential administration of a solid or liquid oral pharmaceutical dosage form containing Compound A, and a further active agent or agents, known to be useful in the treatment of cancer, including chemotherapy and radiation treatment. The term further active agent or agents, as used herein, includes any compound or therapeutic agent known to or that demonstrates advantageous properties when administered to a patient in need of treatment for cancer. As used herein, "further active agent or agents" is used interchangeably with further anti-neoplastic agent or agents. Preferably, if the administration is not simultaneous, the compounds are administered in a close time proximity to each other. Furthermore, it does not matter if the compounds are administered in the same dosage form, e.g. one compound may be administered by injection and another compound may be administered orally. Suitably, the "co-administration" will consist essentially of a liquid oral pharmaceutical dosage form containing compound A and a second pharmaceutical dosage form containing a further active agent. Suitably, the "co-administration" will consist essentially of a liquid oral pharmaceutical dosage form containing compound A, a second pharmaceutical dosage form containing a further active agent, and a third pharmaceutical dosage form containing another further active agent.

Typically, any anti-neoplastic agent that has activity versus a susceptible tumor being treated may be co-administered in the treatment of cancer in the present invention. Examples of such agents can be found in Cancer Principles and Practice of Oncology by V.T. Devita and S. Hellman (editors), 6th edition (February 15, 2001), Lippincott Williams & Wilkins Publishers. A person of ordinary skill in the art would be able to discern which combinations of agents would be useful based on the particular characteristics of the drugs and the cancer involved. Typical anti-neoplastic agents useful in the present invention include, but are not limited to, anti-microtubule agents such as diterpenoids and vinca alkaloids; platinum coordination complexes; alkylating agents such as nitrogen mustards, oxazaphosphorines, alkylsulfonates, nitrosoureas, and triazenes; antibiotic agents such as anthracyclins, actinomycins and bleomycins; topoisomerase II inhibitors such as epipodophyllotoxins; antimetabolites such as purine and pyrimidine analogues and anti-folate compounds; topoisomerase I inhibitors such as camptothecins; hormones and hormonal analogues; signal transduction pathway inhibitors; non-receptor tyrosine kinase angiogenesis inhibitors; immunotherapeutic agents; proapoptotic agents; cell cycle signaling inhibitors; proteasome inhibitors; and inhibitors of cancer metabolism.

Examples of a further active agent or agents (anti-neoplastic agent) for use in combination or co-administered with a presently invented pharmaceutical dosage form, are chemotherapeutic agents.

Anti-microtubule or anti-mitotic agents are phase specific agents active against the microtubules of tumor cells during M or the mitosis phase of the cell cycle. Examples of anti-microtubule agents include, but are not limited to, diterpenoids and vinca alkaloids.

Diterpenoids, which are derived from natural sources, are phase specific anti-cancer agents that operate at the G₂/M phases of the cell cycle. It is believed that the diterpenoids stabilize the β-tubulin subunit of the microtubules, by binding with this protein. Disassembly of the protein appears then to be inhibited with mitosis being arrested and cell death following. Examples of diterpenoids include, but are not limited to, paclitaxel and its analog docetaxel.

Paclitaxel, 5β,20-epoxy-1,2α,4,7β,10β, 13α-hexa-hydroxytax-11-en-9-one 4,10-diacetate 2-benzoate 13-ester with (2R,3S)-N-benzoyl-3-phenylisoserine; is a natural diterpene product isolated from the Pacific yew tree *Taxus brevifolia* and is commercially available as an injectable solution TAXOL®. It is a member of the taxane family of terpenes. Paclitaxel has been approved for clinical use in the treatment of refractory ovarian cancer and breast cancer in the United States.

Docetaxel, (2R,3S)- N-carboxy-3-phenylisoserine,N-*tert*-butyl ester, 13-ester with 5β-20-epoxy-1,2α,4,7β,10β,13α-hexahydroxytax-11-en-9-one 4-acetate 2-benzoate, trihydrate; is commercially available as an injectable solution as TAXOTERE®. Docetaxel is indicated for the treatment of breast cancer. Docetaxel is a semisynthetic derivative of paclitaxel *q.v.,* prepared using a natural precursor, 10-deacetyl-baccatin III, extracted from the needle of the European Yew tree. The dose limiting toxicity of docetaxel is neutropenia.

Vinca alkaloids are phase specific anti-neoplastic agents derived from the periwinkle plant. Vinca alkaloids act at the M phase (mitosis) of the cell cycle by binding specifically to tubulin. Consequently, the bound tubulin molecule is unable to polymerize into microtubules. Mitosis is believed to be arrested in metaphase with cell death following. Examples of vinca alkaloids include, but are not limited to, vinblastine, vincristine, and vinorelbine.

Vinblastine, vincaleukoblastine sulfate, is commercially available as VELBAN® as an injectable solution. Although, it has possible indication as a second line therapy of various solid tumors, it is primarily indicated in the treatment of testicular cancer and various lymphomas including Hodgkin's Disease; and lymphocytic and histiocytic lymphomas. Myelosuppression is the dose limiting side effect of vinblastine.

Vincristine, vincaleukoblastine, 22-oxo-, sulfate, is commercially available as ONCOVIN® as an injectable solution. Vincristine is indicated for the treatment of acute leukemias and has also found use in treatment regimens for Hodgkin's and non-Hodgkin's malignant lymphomas. Alopecia and neurologic effects are the most common side effect of vincristine and to a lesser extent myelosupression and gastrointestinal mucositis effects occur.

Vinorelbine, 3',4'-didehydro -4'-deoxy-C'-norvincaleukoblastine [R-(R*,R*)-2,3-dihydroxybutanedioate (1:2)(salt)], commercially available as an injectable solution of vinorelbine tartrate (NAVELBINE®), is a semisynthetic vinca alkaloid. Vinorelbine is indicated as a single agent or in combination with other chemotherapeutic agents, such as cisplatin, in the treatment of various solid tumors, particularly non-small cell lung, advanced breast, and hormone refractory prostate cancers. Myelosuppression is the most common dose limiting side effect of vinorelbine.

Platinum coordination complexes are non-phase specific anti-cancer agents, which are interactive with DNA. The platinum complexes enter tumor cells, undergo, aquation and form intra- and interstrand crosslinks with DNA causing adverse biological effects to the tumor. Examples of platinum coordination complexes include, but are not limited to, cisplatin and carboplatin.

Cisplatin, cis-diamminedichloroplatinum, is commercially available as PLATlNOL® as an injectable solution. Cisplatin is primarily indicated in the treatment of metastatic testicular and ovarian cancer and advanced bladder cancer. The primary dose limiting side effects of cisplatin are nephrotoxicity, which may be controlled by hydration and diuresis, and ototoxicity.

Carboplatin, platinum, diammine [1,1-cyclobutane-dicarboxylate(2-)-O,O'], is commercially available as PARAPLATIN® as an injectable solution. Carboplatin is primarily indicated in the first and second line treatment of advanced ovarian carcinoma. Bone marrow suppression is the dose limiting toxicity of carboplatin.

Alkylating agents are non-phase anti-cancer specific agents and strong electrophiles. Typically, alkylating agents form covalent linkages, by alkylation, to DNA through nucleophilic moieties of the DNA molecule such as phosphate, amino, sulfhydryl, hydroxyl, carboxyl, and imidazole groups. Such alkylation disrupts nucleic acid function leading to cell death. Examples of alkylating agents include, but are not limited to, nitrogen mustards such as cyclophosphamide, melphalan, and chlorambucil; alkyl sulfonates such as busulfan; nitrosoureas such as carmustine; and triazenes such as dacarbazine.

Cyclophosphamide, 2-[bis(2-chloroethyl)amino]tetrahydro-2H-1,3,2-oxazaphosphorine 2-oxide monohydrate, is commercially available as an injectable solution or tablets as CYTOXAN®. Cyclophosphamide is indicated as a single agent or in combination with other chemotherapeutic agents, in the treatment of malignant lymphomas, multiple myeloma, and leukemias. Alopecia, nausea, vomiting and leukopenia are the most common dose limiting side effects of cyclophosphamide.

Melphalan, 4-[bis(2-chloroethyl)amino]-L-phenylalanine, is commercially available as an injectable solution or tablets as ALKERAN®. Melphalan is indicated for the palliative treatment of multiple myeloma and non-resectable epithelial carcinoma of the ovary. Bone marrow suppression is the most common dose limiting side effect of melphalan.

Chlorambucil, 4-[bis(2-chloroethyl)amino]benzenebutanoic acid, is commercially available as LEUKERAN® tablets. Chlorambucil is indicated for the palliative treatment of chronic lymphatic leukemia, and malignant lymphomas such as lymphosarcoma, giant follicular lymphoma, and Hodgkin's disease. Bone marrow suppression is the most common dose limiting side effect of chlorambucil.

Busulfan, 1,4-butanediol dimethanesulfonate, is commercially available as MYLERAN® TABLETS. Busulfan is indicated for the palliative treatment of chronic myelogenous leukemia. Bone marrow suppression is the most common dose limiting side effects of busulfan.

Carmustine, 1,3-[bis(2-chloroethyl)-1-nitrosourea, is commercially available as single vials of lyophilized material as BiCNU®. Carmustine is indicated for the palliative treatment as a single agent or in combination with other agents for brain tumors, multiple myeloma, Hodgkin's disease, and non-Hodgkin's lymphomas. Delayed myelosuppression is the most common dose limiting side effects of carmustine.

Dacarbazine, 5-(3,3-dimethyl-1-triazeno)-imidazole-4-carboxamide, is commercially available as single vials of material as DTIC-Dome®. Dacarbazine is indicated for the treatment of metastatic malignant melanoma and in combination with other agents for the second line treatment of Hodgkin's Disease. Nausea, vomiting, and anorexia are the most common dose limiting side effects of dacarbazine.

Antibiotic anti-neoplastics are non-phase specific agents, which bind or intercalate with DNA. Typically, such action results in stable DNA complexes or strand breakage, which disrupts ordinary function of the nucleic acids leading to cell death. Examples of antibiotic anti-neoplastic agents include, but are not limited to, actinomycins such as dactinomycin, anthrocyclins such as daunorubicin and doxorubicin; and bleomycins.

Dactinomycin, also known as Actinomycin D, is commercially available in injectable form as COSMEGEN®. Dactinomycin is indicated for the treatment of Wilm's tumor and rhabdomyosarcoma. Nausea, vomiting, and anorexia are the most common dose limiting side effects of dactinomycin.

Daunorubicin, (8S-cis-)-8-acetyl-10-[(3-amino-2,3,6-trideoxy-α-L-lyxo-hexopyranosyl)oxy]-7,8,9,10-tetrahydro-6,8,11-trihydroxy-1-methoxy-5,12 naphthacenedione hydrochloride, is commercially available as a liposomal injectable form as DAUNOXOME® or as an injectable as CERUBIDINE®. Daunorubicin is indicated for remission induction in the treatment of acute nonlymphocytic leukemia and advanced HIV associated Kaposi's sarcoma. Myelosuppression is the most common dose limiting side effect of daunorubicin.

Doxorubicin, (8S, 10S)-10-[(3-amino-2,3,6-trideoxy-α-L-lyxo-hexopyranosyl)oxy]-8-glycoloyl, 7,8,9,10-tetrahydro-6,8,11-trihydroxy-1-methoxy-5,12 naphthacenedione hydrochloride, is commercially available as an injectable form as RUBEX® or ADRIAMYCIN RDF®. Doxorubicin is primarily indicated for the treatment of acute lymphoblastic leukemia and acute myeloblastic leukemia, but is also a useful component in the treatment of some solid tumors and lymphomas. Myelosuppression is the most common dose limiting side effect of doxorubicin.

Bleomycin, a mixture of cytotoxic glycopeptide antibiotics isolated from a strain of *Streptomyces verticillus,* is commercially available as BLENOXANE®. Bleomycin is indicated as a palliative treatment, as a single agent or in combination with other agents, of squamous cell carcinoma, lymphomas, and testicular carcinomas. Pulmonary and cutaneous toxicities are the most common dose limiting side effects of bleomycin.

Topoisomerase II inhibitors include, but are not limited to, epipodophyllotoxins.

Epipodophyllotoxins are phase specific anti-neoplastic agents derived from the mandrake plant. Epipodophyllotoxins typically affect cells in the S and G₂ phases of the cell cycle by forming a ternary complex with topoisomerase II and DNA causing DNA strand breaks. The strand breaks accumulate and cell death follows. Examples of epipodophyllotoxins include, but are not limited to, etoposide and teniposide.

Etoposide, 4'-demethyl-epipodophyllotoxin 9[4,6-0-(R)-ethylidene-β-D-glucopyranoside], is commercially available as an injectable solution or capsules as VePESID® and is commonly known as VP-16. Etoposide is indicated as a single agent or in combination with other chemotherapy agents in the treatment of testicular and non-small cell lung cancers. Myelosuppression is the most common side effect of etoposide. The incidence of leucopenia tends to be more severe than thrombocytopenia.

Teniposide, 4'-demethyl-epipodophyllotoxin 9[4,6-0-(R)-thenylidene-β-D-glucopyranoside], is commercially available as an injectable solution as VUMON® and is commonly known as VM-26. Teniposide is indicated as a single agent or in combination with other chemotherapy agents in the treatment of acute leukemia in children. Myelosuppression is the most common dose limiting side effect of teniposide. Teniposide can induce both leucopenia and thrombocytopenia.

Antimetabolite neoplastic agents are phase specific anti-neoplastic agents that act at S phase (DNA synthesis) of the cell cycle by inhibiting DNA synthesis or by inhibiting purine or pyrimidine base synthesis and thereby limiting DNA synthesis. Consequently, S phase does not proceed and cell death follows. Examples of antimetabolite anti-neoplastic agents include, but are not limited to, fluorouracil, methotrexate, cytarabine, mecaptopurine, thioguanine, and gemcitabine.

5-fluorouracil, 5-fluoro-2,4- (1H,3H) pyrimidinedione, is commercially available as fluorouracil. Administration of 5-fluorouracil leads to inhibition of thymidylate synthesis and is also incorporated into both RNA and DNA. The result typically is cell death. 5-fluorouracil is indicated as a single agent or in combination with other chemotherapy agents in the treatment of carcinomas of the breast, colon, rectum, stomach and pancreas. Myelosuppression and mucositis are dose limiting side effects of 5-fluorouracil. Other fluoropyrimidine analogs include 5-fluoro deoxyuridine (floxuridine) and 5-fluorodeoxyuridine monophosphate.

Cytarabine, 4-amino-1-β-D-arabinofuranosyl-2 (1H)-pyrimidinone, is commercially available as CYTOSAR-U® and is commonly known as Ara-C. It is believed that cytarabine exhibits cell phase specificity at S-phase by inhibiting DNA chain elongation by terminal incorporation of cytarabine into the growing DNA chain. Cytarabine is indicated as a single agent or in combination with other chemotherapy agents in the treatment of acute leukemia. Other cytidine analogs include 5-azacytidine and 2',2'-difluorodeoxycytidine (gemcitabine). Cytarabine induces leucopenia, thrombocytopenia, and mucositis.

Mercaptopurine, 1,7-dihydro-6H-purine-6-thione monohydrate, is commercially available as PURINETHOL®. Mercaptopurine exhibits cell phase specificity at S-phase by inhibiting DNA synthesis by an as of yet unspecified mechanism. Mercaptopurine is indicated as a single agent or in combination with other chemotherapy agents in the treatment of acute leukemia. Myelosuppression and gastrointestinal mucositis are expected side effects of mercaptopurine at high doses. A useful mercaptopurine analog is azathioprine.

Thioguanine, 2-amino-1,7-dihydro-6H-purine-6-thione, is commercially available as TABLOID®. Thioguanine exhibits cell phase specificity at S-phase by inhibiting DNA synthesis by an as of yet unspecified mechanism. Thioguanine is indicated as a single agent or in combination with other chemotherapy agents in the treatment of acute leukemia. Myelosuppression, including leucopenia, thrombocytopenia, and anemia, is the most common dose limiting side effect of thioguanine administration. However, gastrointestinal side effects occur and can be dose limiting. Other purine analogs include pentostatin, erythrohydroxynonyladenine, fludarabine phosphate, and cladribine.

Gemcitabine, 2'-deoxy-2', 2'-difluorocytidine monohydrochloride (β-isomer), is commercially available as GEMZAR®. Gemcitabine exhibits cell phase specificity at S-phase and by blocking progression of cells through the G1/S boundary. Gemcitabine is indicated in combination with cisplatin in the treatment of locally advanced non-small cell lung cancer and alone in the treatment of locally advanced pancreatic cancer. Myelosuppression, including leucopenia, thrombocytopenia, and anemia, is the most common dose limiting side effect of gemcitabine administration.

Methotrexate, N-[4[[(2,4-diamino-6-pteridinyl) methyl]methylamino] benzoyl]-L-glutamic acid, is commercially available as methotrexate sodium. Methotrexate exhibits cell phase effects specifically at S-phase by inhibiting DNA synthesis, repair and/or replication through the inhibition of dyhydrofolic acid reductase which is required for synthesis of purine nucleotides and thymidylate. Methotrexate is indicated as a single agent or in combination with other chemotherapy agents in the treatment of choriocarcinoma, meningeal leukemia, non-Hodgkin's lymphoma, and carcinomas of the breast, head, neck, ovary and bladder. Myelosuppression (leucopenia, thrombocytopenia, and anemia) and mucositis are expected side effect of methotrexate administration.

Camptothecins, including, camptothecin and camptothecin derivatives are available or under development as Topoisomerase I inhibitors. Camptothecins cytotoxic activity is believed to be related to its Topoisomerase I inhibitory activity. Examples of camptothecins include, but are not limited to irinotecan, topotecan, and the various optical forms of 7-(4-methylpiperazino-methylene)-10,11-ethylenedioxy-20-camptothecin described below.

Irinotecan HCI, (4S)-4,11-diethyl-4-hydroxy-9-[(4-piperidinopiperidino) carbonyloxy]-1H-pyrano[3',4',6,7]indolizino[1,2-b]quinoline-3,14(4H,12H)-dione hydrochloride, is commercially available as the injectable solution CAMPTOSAR®.

Irinotecan is a derivative of camptothecin which binds, along with its active metabolite SN-38, to the topoisomerase I - DNA complex. It is believed that cytotoxicity occurs as a result of irreparable double strand breaks caused by interaction of the topoisomerase I : DNA : irintecan or SN-38 ternary complex with replication enzymes. Irinotecan is indicated for treatment of metastatic cancer of the colon or rectum. The dose limiting side effects of irinotecan HCI are myelosuppression, including neutropenia, and GI effects, including diarrhea.

Topotecan HCI, (S)-10-[(dimethylamino)methyl]-4-ethyl-4,9-dihydroxy-1H-pyrano[3',4',6,7]indolizino[1,2-b]quinoline-3,14-(4H,12H)-dione monohydrochloride, is commercially available as the injectable solution HYCAMTIN®. Topotecan is a derivative of camptothecin which binds to the topoisomerase I - DNA complex and prevents religation of singles strand breaks caused by Topoisomerase I in response to torsional strain of the DNA molecule. Topotecan is indicated for second line treatment of metastatic carcinoma of the ovary and small cell lung cancer. The dose limiting side effect of topotecan HCI is myelosuppression, primarily neutropenia.

Also of interest, is the camptothecin derivative of Formula A following, including the racemic mixture (R,S) form as well as the R and S enantiomers:

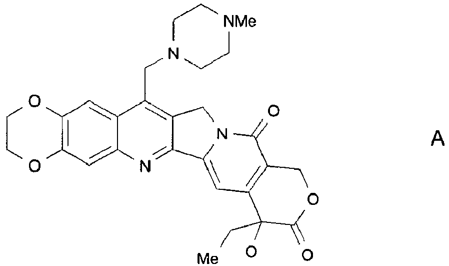

known by the chemical name "7-(4-methylpiperazino-methylene)-10,11-ethylenedioxy-20(R,S)-camptothecin (racemic mixture) or "7-(4-methylpiperazino-methylene)-10,11-ethylenedioxy-20(R)-camptothecin (R enantiomer) or "7-(4-methylpiperazino-methylene)-10,11-ethylenedioxy-20(S)-camptothecin (S enantiomer). Such compound as well as related compounds are described, including methods of making, in U.S. Patent Nos. 6,063,923; 5,342,947; 5,559,235; and 5,491,237.

Hormones and hormonal analogues are useful compounds for treating cancers in which there is a relationship between the hormone(s) and growth and/or lack of growth of the cancer. Examples of hormones and hormonal analogues useful in cancer treatment include, but are not limited to, adrenocorticosteroids such as prednisone and prednisolone which are useful in the treatment of malignant lymphoma and acute leukemia in children; aminoglutethimide and other aromatase inhibitors such as anastrozole, letrazole, vorazole, and exemestane useful in the treatment of adrenocortical carcinoma and hormone dependent breast carcinoma containing estrogen receptors; progestrins such as megestrol acetate useful in the treatment of hormone dependent breast cancer and endometrial carcinoma; estrogens, androgens, and anti-androgens such as flutamide, nilutamide, bicalutamide, cyproterone acetate and 5α-reductases such as finasteride and dutasteride, useful in the treatment of prostatic carcinoma and benign prostatic hypertrophy; anti-estrogens such as tamoxifen, toremifene, raloxifene, droloxifene, iodoxyfene, as well as selective estrogen receptor modulators (SERMS) such those described in U.S. Patent Nos. 5,681,835; 5,877,219; and 6,207,716, useful in the treatment of hormone dependent breast carcinoma and other susceptible cancers; and gonadotropin-releasing hormone (GnRH) and analogues thereof which stimulate the release of leutinizing hormone (LH) and/or follicle stimulating hormone (FSH) for the treatment prostatic carcinoma, for instance, LHRH agonists and antagagonists such as goserelin acetate and luprolide.

Signal transduction pathway inhibitors are those inhibitors, which block or inhibit a chemical process which evokes an intracellular change. As used herein this change is cell proliferation or differentiation. Signal tranduction inhibitors useful in the present invention include inhibitors of receptor tyrosine kinases, non-receptor tyrosine kinases, SH2/SH3 domain blockers, serine/threonine kinases, phosphotidylinositol-3 kinases, myo-inositol signaling, and Ras oncogenes.

Several protein tyrosine kinases catalyse the phosphorylation of specific tyrosyl residues in various proteins involved in the regulation of cell growth. Such protein tyrosine kinases can be broadly classified as receptor or non-receptor kinases.

Receptor tyrosine kinases are transmembrane proteins having an extracellular ligand binding domain, a transmembrane domain, and a tyrosine kinase domain. Receptor tyrosine kinases are involved in the regulation of cell growth and are generally termed growth factor receptors. Inappropriate or uncontrolled activation of many of these kinases, i.e. aberrant kinase growth factor receptor activity, for example by over-expression or mutation, has been shown to result in uncontrolled cell growth. Accordingly, the aberrant activity of such kinases has been linked to malignant tissue growth. Consequently, inhibitors of such kinases could provide cancer treatment methods. Growth factor receptors include, for example, epidermal growth factor receptor (EGFr), platelet derived growth factor receptor (PDGFr), erbB2, erbB4, vascular endothelial growth factor receptor (VEGFr), tyrosine kinase with immunoglobulin-like and epidermal growth factor homology domains (TIE-2), insulin growth factor-I (IGFI) receptor, macrophage colony stimulating factor (cfms), BTK, ckit, cmet, fibroblast growth factor (FGF) receptors, Trk receptors (TrkA, TrkB, and TrkC), ephrin (eph) receptors, and the RET protooncogene. Several inhibitors of growth receptors are under development and include ligand antagonists, antibodies, tyrosine kinase inhibitors and anti-sense oligonucleotides. Growth factor receptors and agents that inhibit growth factor receptor function are described, for instance, in Kath, John C., Exp. Opin. Ther. Patents (2000) 10(6):803-818; Shawver et al DDT Vol 2, No. 2 February 1997; and Lofts, F. J. et al, "Growth factor receptors as targets", New Molecular Targets for Cancer Chemotherapy, ed. Workman, Paul and Kerr, David, CRC press 1994, London.

Tyrosine kinases, which are not growth factor receptor kinases are termed non-receptor tyrosine kinases. Non-receptor tyrosine kinases for use in the present invention, which are targets or potential targets of anti-cancer drugs, include cSrc, Lck, Fyn, Yes, Jak, cAbl, FAK (Focal adhesion kinase), Brutons tyrosine kinase, and Bcr-Abl. Such non-receptor kinases and agents which inhibit non-receptor tyrosine kinase function are described in Sinh, S. and Corey, S.J., (1999) Journal of Hematotherapy and Stem Cell Research 8 (5): 465 - 80; and Bolen, J.B., Brugge, J.S., (1997) Annual review of Immunology. 15: 371-404.

SH2/SH3 domain blockers are agents that disrupt SH2 or SH3 domain binding in a variety of enzymes or adaptor proteins including, PI3-K p85 subunit, Src family kinases, adaptor molecules (Shc, Crk, Nck, Grb2) and Ras-GAP. SH2/SH3 domains as targets for anti-cancer drugs are discussed in Smithgall, T.E. (1995), Journal of Pharmacological and Toxicological Methods. 34(3) 125-32.

Inhibitors of Serine/Threonine Kinases including MAP kinase cascade blockers which include blockers of Raf kinases (rafk), Mitogen or Extracellular Regulated Kinase (MEKs), and Extracellular Regulated Kinases (ERKs); and Protein kinase C family member blockers including blockers of PKCs (alpha, beta, gamma, epsilon, mu, lambda, iota, zeta). IkB kinase family (IKKa, IKKb), PKB family kinases, akt kinase family members, PDK1 and TGF beta receptor kinases. Such Serine/Threonine kinases and inhibitors thereof are described in Yamamoto, T., Taya, S., Kaibuchi, K., (1999), Journal of Biochemistry. 126 (5) 799-803; Brodt, P, Samani, A., and Navab, R. (2000), Biochemical Pharmacology, 60. 1101-1107; Massague, J., Weis-Garcia, F. (1996) Cancer Surveys. 27:41-64; Philip, P.A., and Harris, A.L. (1995), Cancer Treatment and Research. 78: 3-27, Lackey, K. et al Bioorganic and Medicinal Chemistry Letters, (10), 2000, 223-226; U.S. Patent No. 6,268,391; Pearce, L.R et al. Nature Reviews Molecular Cell Biology (2010) 11, 9-22. and Martinez-lacaci, L., et al, Int. J. Cancer (2000), 88(1), 44-52.

Suitably, the pharmaceutically active compound of the invention is used in combination with a B-Raf inhibitor. Suitably, *N*-{3-[5-(2-Amino-4-pyrimidinyl)-2-(1,1-dimethylethyl)-1,3-thiazol-4-yl]-2-fluorophenyl}-2,6-difluorobenzenesulfonamide, or a pharmaceutically acceptable salt thereof, which is disclosed and claimed, in International Application No. PCT/US2009/042682, having an International filing date of May 4, 2009, the entire disclosure of which is hereby incorporated by reference. *N*-{3-[5-(2-Amino-4-pyrimidinyl)-2-(1,1-dimethylethyl)-1,3-thiazol-4-yl]-2-fluorophenyl}-2,6-difluorobenzenesulfonamide can be prepared as described in International Application No. PCT/US2009/042682.

Suitably, the pharmaceutically active compound of the invention is used in combination with an Akt inhibitor. Suitably, N-{(1S)-2-amino-1-[(3,4-difluorophenyl)methyl]ethyl}-5-chloro-4-(4-chloro-1-methyl-1H-pyrazol-5-yl)-2-furancarboxamide or a pharmaceutically acceptable salt thereof, which is disclosed and claimed in International Application No. PCT/US2008/053269, having an International filing date of February 7, 2008; International Publication Number WO 2008/098104 and an International Publication date of August 14, 2008, the entire disclosure of which is hereby incorporated by reference. N-{(1S)-2-amino-1-[(3,4-difluorophenyl)methyl]ethyl}-5-chloro-4-(4-chloro-1-methyl-1H-pyrazol-5-yl)-2-furancarboxamide is the compound of example 224 and can be prepared as described in International Application No. PCT/US2008/053269.

Suitably, the pharmaceutically active compound of the invention is used in combination with an Akt inhibitor. Suitably, *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide or a pharmaceutically acceptable salt thereof, which is disclosed and claimed in International Application No. PCT/US2008/053269, having an International filing date of February 7, 2008; International Publication Number WO 2008/098104 and an International Publication date of August 14, 2008, the entire disclosure of which is hereby incorporated by reference. *N*-{(1S)-2-amino-1-[(3-fluorophenyl)methyl]ethyl}-5-chloro-4-(4-chloro-1-methyl-1*H*-pyrazol-5-yl)-2-thiophenecarboxamide is the compound of example 96 and can be prepared as described in International Application No. PCT/US2008/053269. Suitably, *N*-{(1*S*)-2-amino-1-[(3-fluorophenyl)methyl]ethyl}-5-chloro-4-(4-chloro-1-methyl-1*H*-pyrazol-5-yl)-2-thiophenecarboxamide is in the form of a hydrochloride salt. The salt form can be prepared by one of skill in the art from the description in International Application No. PCT/US2010/022323, having an International filing date of January 28, 2010.

Inhibitors of Phosphotidylinositol-3 Kinase family members including blockers of PI3-kinase, ATM, DNA-PK, and Ku may also be useful in the present invention. Such kinases are discussed in Abraham, R.T. (1996), Current Opinion in Immunology. 8 (3) 412-8; Canman, C.E., Lim, D.S. (1998), Oncogene 17 (25) 3301-3308; Jackson, S.P. (1997), International Journal of Biochemistry and Cell Biology. 29 (7):935-8; and Zhong, H. et al, Cancer res, (2000) 60(6), 1541-1545.

Also of interest in the present invention are Myo-inositol signaling inhibitors such as phospholipase C blockers and Myoinositol analogues. Such signal inhibitors are described in Powis, G., and Kozikowski A., (1994) New Molecular Targets for Cancer Chemotherapy ed., Paul Workman and David Kerr, CRC press 1994, London.

Another group of signal transduction pathway inhibitors are inhibitors of Ras Oncogene. Such inhibitors include inhibitors of farnesyltransferase, geranyl-geranyl transferase, and CAAX proteases as well as anti-sense oligonucleotides, ribozymes and immunotherapy. Such inhibitors have been shown to block ras activation in cells containing wild type mutant ras, thereby acting as antiproliferation agents. Ras oncogene inhibition is discussed in Scharovsky, O.G., Rozados, V.R., Gervasoni, S.I. Matar, P. (2000), Journal of Biomedical Science. 7(4) 292-8; Ashby, M.N. (1998), Current Opinion in Lipidology. 9 (2) 99 - 102; and BioChim. Biophys. Acta, (19899) 1423(3):19-30.

As mentioned above, antibody antagonists to receptor kinase ligand binding may also serve as signal transduction inhibitors. This group of signal transduction pathway inhibitors includes the use of humanized antibodies to the extracellular ligand binding domain of receptor tyrosine kinases. For example Imclone C225 EGFR specific antibody (see Green, M.C. et al, Monoclonal Antibody Therapy for Solid Tumors, Cancer Treat. Rev., (2000), 26(4), 269-286); Herceptin ® erbB2 antibody; and 2CB VEGFR2 specific antibody (see Brekken, R.A. et al, Selective Inhibition of VEGFR2 Activity by a monoclonal Anti-VEGF antibody blocks tumor growth in mice, Cancer Res. (2000) 60, 5117-5124).

Non-receptor kinase angiogenesis inhibitors may also be useful in the present invention. Inhibitors of angiogenesis related VEGFR and TIE2 are discussed above in regard to signal transduction inhibitors (both receptors are receptor tyrosine kinases). Angiogenesis in general is linked to erbB2/EGFR signaling since inhibitors of erbB2 and EGFR have been shown to inhibit angiogenesis, primarily VEGF expression. Accordingly, non-receptor tyrosine kinase inhibitors may be used in combination with the compounds of the present invention. For example, anti-VEGF antibodies, which do not recognize VEGFR (the receptor tyrosine kinase), but bind to the ligand; small molecule inhibitors of integrin (alphaᵥ beta₃) that will inhibit angiogenesis; endostatin and angiostatin (non-RTK) may also prove useful in combination with the disclosed compounds.

Agents used in immunotherapeutic regimens may also be useful in combination with the compounds of Formula (I). There are a number of immunologic strategies to generate an immune response. These strategies are generally in the realm of tumor vaccinations. The efficacy of immunologic approaches may be greatly enhanced through combined inhibition of signaling pathways using a small molecule inhibitor. Discussion of the immunologic/tumor vaccine approach against erbB2/EGFR are found in Reilly RT et al. (2000), Cancer Res. 60: 3569-3576; and Chen Y, Hu D, Eling DJ, Robbins J, and Kipps TJ. (1998), Cancer Res. 58: 1965-1971.

Agents used in proapoptotic regimens (e.g., bcl-2 antisense oligonucleotides) may also be used in the combination of the present invention. Members of the Bcl-2 family of proteins block apoptosis. Upregulation of bcl-2 has therefore been linked to chemoresistance. Studies have shown that the epidermal growth factor (EGF) stimulates anti-apoptotic members of the bcl-2 family (i.e., mcl-1). Therefore, strategies designed to downregulate the expression of bcl-2 in tumors have demonstrated clinical benefit, namely Genta's G3139 bcl-2 antisense oligonucleotide. Such proapoptotic strategies using the antisense oligonucleotide strategy for bcl-2 are discussed in Water JS et al. (2000), J. Clin. Oncol. 18: 1812-1823; and Kitada S et al. (1994), Antisense Res. Dev. 4: 71-79.

Cell cycle signalling inhibitors inhibit molecules involved in the control of the cell cycle. A family of protein kinases called cyclin dependent kinases (CDKs) and their interaction with a family of proteins termed cyclins controls progression through the eukaryotic cell cycle. The coordinate activation and inactivation of different cyclin/CDK complexes is necessary for normal progression through the cell cycle. Several inhibitors of cell cycle signalling are under development. For instance, examples of cyclin dependent kinases, including CDK2, CDK4, and CDK6 and inhibitors for the same are described in, for instance, Rosania et al, Exp. Opin. Ther. Patents (2000) 10(2):215-230. Further, p21WAF1/CIP1 has been described as a potent and universal inhibitor of cyclin-dependent kinases (Cdks) (Ball et al., Progress in Cell Cycle Res., 3: 125 (1997)). Compounds that are known to induce expression of p21WAF1/CIP1 have been implicated in the suppression of cell proliferation and as having tumor suppressing activity (Richon et al., Proc. Nat Acad. Sci. U.S.A. 97(18): 10014-10019 (2000)), and are included as cell cycle signaling inhibitors. Histone deacetylase (HDAC) inhibitors are implicated in the transcriptional activation of p21WAF1/CIP1 (Vigushin et al., Anticancer Drugs, 13(1): 1-13 (Jan 2002)), and are suitable cell cycle signaling inhibitors for use herein.

Examples of such HDAC inhibitors include:
1.Vorinostat, including pharmaceutically acceptable salts thereof. Marks et al., Nature Biotechnology 25, 84 to 90 (2007); Stenger, Community Oncology 4, 384-386 (2007).
   Vorinostat has the following chemical structure and name: 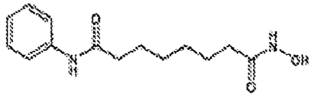 *N*-hydroxy-*N'*-phenyl-octanediamide
2. Romidepsin, including pharmaceutically acceptable salts thereof. Vinodhkumar et al., Biomedicine & Pharmacotherapy 62 (2008) 85-93. Romidepsin, has the following chemical structure and name: 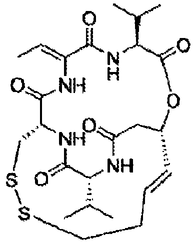 (1S,4S,7Z,10S,16E,21R)-7-ethylidene-4,21-di(propan-2-yl)-2-oxa-12,13-dithia-5,8,20,23-tetrazabicyclo[8.7.6]tricos-16-ene-3,6,9,19,22-pentone
3.Panobinostat, including pharmaceutically acceptable salts thereof. Drugs of the Future 32(4): 315-322 (2007).
   Panobinostat, has the following chemical structure and name: 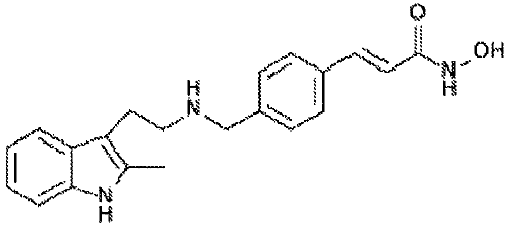 (2*E*)-*N*-hydroxy-3-[4-({[2-(2-methyl-1*H*-indol-3-yl)ethyl]amino}methyl)phenyl]acrylamide
4.Valproic acid, including pharmaceutically acceptable salts thereof. Gottlicher, et al., EMBO J. 20(24): 6969-6978 (2001).
   Valproic acid, has the following chemical structure and name: 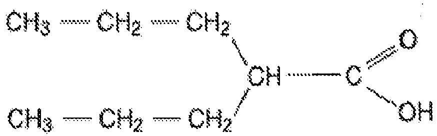 2-propylpentanoic acid
   5.Mocetinostat (MGCD0103), including pharmaceutically acceptable salts thereof. Balasubramanian et al., Cancer Letters 280: 211-221 (2009).
   Mocetinostat, has the following chemical structure and name: 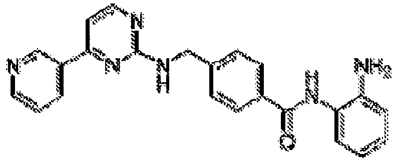 *N*-(2-Aminophenyl)-4-[[(4-pyridin-3-ylpyrimidin-2-yl)amino]methyl] benzamide

Further examples of such HDAC inhibitors are included in Bertrand European Journal of Medicinal Chemistry 45, (2010) 2095-2116, particularly the compounds of table 3 therein as indicated below.

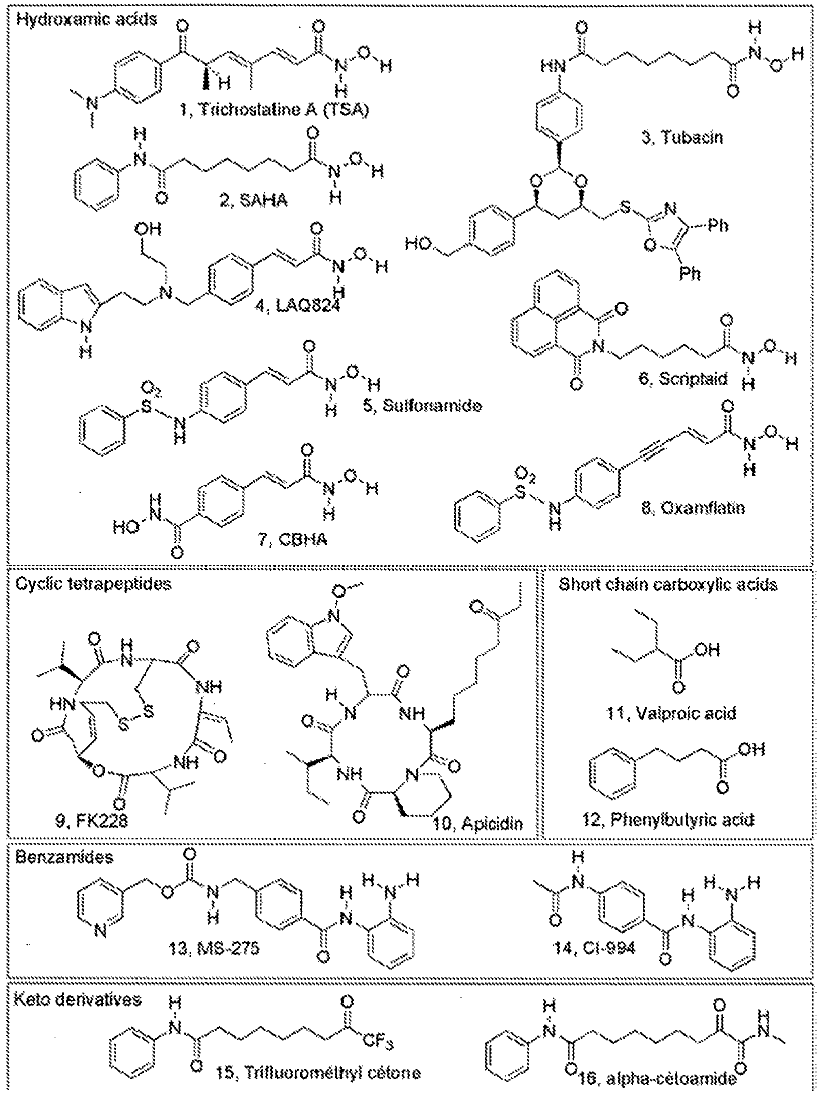

Proteasome inhibitors are drugs that block the action of proteasomes, cellular complexes that break down proteins, like the p53 protein. Several proteasome inhibitors are marketed or are being studied in the treatment of cancer. Suitable proteasome inhibitors for use herein include:
1.Bortezomib (Velcade®), including pharmaceutically acceptable salts thereof. Adams J, Kauffman M (2004), Cancer Invest 22 (2): 304-11.
   Bortezomib has the following chemical structure and name. 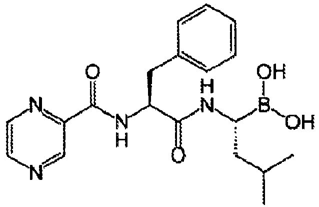 [(1*R*)-3-methyl-1-({(2*S*)-3-phenyl-2-[(pyrazin-2-ylcarbonyl)amino]propanoyl}amino)butyl]boronic acid
2. Disulfiram, including pharmaceutically acceptable salts thereof.
   Bouma et al. (1998). J. Antimicrob. Chemother. 42 (6): 817-20.
   Disulfiram has the following chemical structure and name. 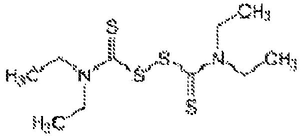 1,1',1", 1'''-[disulfanediylbis(carbonothioylnitrilo)]tetraethane
3.Epigallocatechin gallate (EGCG), including pharmaceutically acceptable salts thereof. Williamson et al., (December 2006), The Journal of Allergy and Clinical Immunology 118 (6): 1369-74.
   Epigallocatechin gallate has the following chemical structure and name. 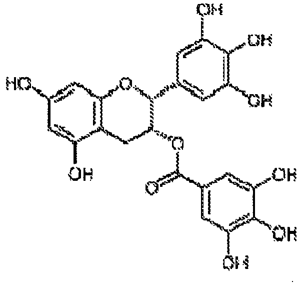 [(2*R*,3*R*)-5,7-dihydroxy-2-(3,4,5-trihydroxyphenyl)chroman-3-yl]3,4,5-trihydroxybenzoate
4.Salinosporamide A, including pharmaceutically acceptable salts thereof. Feling et at., (2003), Angew. Chem. Int. Ed. Engl. 42 (3): 355-7.
   Salinosporamide A has the following chemical structure and name. 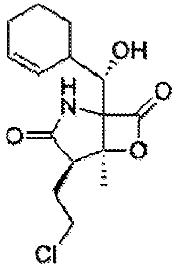 (4R,5S)-4-(2-chloroethyl)-1-((1S)-cyclohex-2-enyl(hydroxy)methyl) -5-methyl-6-oxa-2-azabicyclo3.2.0heptane-3,7-dione

Inhibitors of cancer metabolism - Many tumor cells show a markedly different metabolism from that of normal tissues. For example, the rate of glycolysis, the metabolic process that converts glucose to pyruvate, is increased, and the pyruvate generated is reduced to lactate, rather than being further oxidized in the mitochondria via the tricarboxylic acid (TCA) cycle. This effect is often seen even under aerobic conditions and is known as the Warburg Effect.

Lactate dehydrogenase A (LDH-A), an isoform of lactate dehydrogenase expressed in muscle cells, plays a pivotal role in tumor cell metabolism by performing the reduction of pyruvate to lactate, which can then be exported out of the cell. The enzyme has been shown to be upregulated in many tumor types. The alteration of glucose metabolism described in the Warburg effect is critical for growth and proliferation of cancer cells and knocking down LDH-A using RNA-i has been shown to lead to a reduction in cell proliferation and tumor growth in xenograft models.
D. A. Tennant et. al., Nature Reviews, 2010, 267.
P. Leder, et. al., Cancer Cell, 2006, 9, 425.

Inhibitors of cancer metabolism, including inhibitors of LDH-A, are suitable for use in combination with the formulations of this invention.

Examples of the solubilizers, surfactants, buffers, preservatives, sweeteners, and flavors are understood in the art, such components are generally described, for example, in Martindale--The Extra Pharmacopoeia Pharmaceutical Press, London (1993) and Martin (ed.), Remington's Pharmaceutical Sciences, and the Handbook of Pharmaceutical Excipients.

As used herein, "solubilizer" is a substance (liquid or solid) that helps to keep the drug uniformly dispersed and dissolved in solution. A solubilizer prevents recrystallization and precipitation of the dissolved drug out of solution. For use in the present invention, suitable solubilizers include, but are not limited to, hypromellose, polyvinylpyrrolidone, sulfobutylether B-cyclodextrin (Captisol) and hydroxypropyl B-cyclodextrin (Cavitron). A combination of solubilizers can also be used. For example, hypromellose and hydroxypropyl B-cyclodextrin gave good aqueous solubility of Compound A. Polyethylene glycol, and propylene glycol were found disadvantageous due to the very low aqueous solubility of Compound A in these solubilizers. Suitably, the amount of solubilizer in a formulation according to the invention will be selected from: about 30 to 95%; suitably about 50 to 80%; suitably about 65 to 75%; by weight of the final product. Suitably, the amount of solubilizer in a formulation according to the invention will be selected from: about 30%; suitably about 35%, suitably about 40%, suitably about 45%, suitably about 50%, suitably about 55%, suitably about 60%, suitably about 65%, suitably about 70%, suitably about 75%, suitably about 80%, suitably about 85%, suitably about 90%, and suitably about 95%, by weight of the final product.

As used herein, "surfactant" is a surface active agent that lowers the surface tension of a liquid, the interfacial tension between two liquids, and the interfacial tension between a liquid and a solid, thereby increasing the wettability of the drug particles making it easier to dissolve. For instance, suitable surfactants include, but are not limited to, hypromellose (HPMC), polysorbate 80, polysorbate 20, and sodium lauryl sulphate (SLS). The preferred surfactant is hypromellose because, in this case, it was found to act as both a surfactant and a solubilizer. Suitably, the amount of surfactant in a formulation according to the invention will be selected from: about 0 to 5%; suitably about 0.5 to 4%, suitably about 0.5 to 2%, by weight of the final product. Suitably when present, the amount of surfactant in a formulation according to the invention will be selected from: about 0.5%; suitably about 1%, suitably about 1.5%, by weight of the final product.

As used herein, "buffer" is a mixture of a weak acid and its conjugate base or a weak base and its conjugate acid that is used to resist the change in pH by the addition of a small amount of acid or base. Suitable buffers for use herein include Citric acid monohydrate and Sodium Phosphate, Dibasic, Anhydrous or a combination thereof. Suitably, the amount of total buffer in a formulation according to the invention will be selected from: about 5 to 25%; suitably about 8 to 20%, suitably about 10 to 20%, by weight of the final product. Suitably, the amount of total buffer in a formulation according to the invention will be selected from: about 5%; suitably about 10%, suitably about 15%, suitably about 20%, by weight of the final product.

As used herein, "preservative" is used to prevent the growth of bacteria and/or fungi in the liquid formulation. For instance, suitable preservatives include, but are not limited to, parabens (methyl, ethyl, propyl, and butyl), paraben sodium salt, potassium sorbate, sodium benzoate, and sorbic acid. Suitably, the amount of total preservative in a formulation according to the invention will be selected from: about 0.5 to 4%; suitably about 1 to 3%, suitably about 1 to 2.5%, by weight of the final product. Suitably, the amount of total preservative in a formulation according to the invention will be selected from: about 0.5%; suitably about 1%, suitably about 1.5%, suitably about 2%, by weight of the final product.

As used herein, "sweetener" is a substance (solid or liquid) that is used to improve the palatability of the formulation. For instance, suitable sweeteners include, but are not limited to xylitab, xylitol, mannitol, sucrose, sucralose, saccharin, ammonium and sodium glyceryrhizinate, aspartame, and sorbitol. Suitably, the amount of sweetener in a formulation according to the invention will be selected from: about 5 to 25%; suitably about 8 to 20%, suitably about 10 to 20%, by weight of the final product. Suitably, the amount of sweetener in a formulation according to the invention will be selected from:
about 5%; suitably about 10%, suitably about 15%, suitably about 20%, by weight of the final product.

As used herein, "flavor" is a substance (liquid or solid) that provides a distinct taste and aroma to the formulation. Flavors also help to improve the palatability of the formulation. Suitably the flavor is Strawberry flavor. Suitably, the amount of flavor in a formulation according to the invention will be selected from: about 0.5 to 4%; suitably about 1 to 3%, suitably about 1 to 2.5%, by weight of the final product. Suitably, the amount of flavor in a formulation according to the invention will be selected from: about 0.5%; suitably about 1%, suitably about 1.5%, suitably about 2%, by weight of the final product.

As used herein, "vehicle" is a liquid use to reconstitute a powder into an oral suspension or solution. The vehicle needs to be compatible with the formulation so that stability can be attained and maintained. For instance, suitable vehicles include, but are not limited to, purified water, sterile water for injection, and sterile water for irrigation. According to one embodiment, the vehicle is purified or sterile water.

### Solubility of trametinib

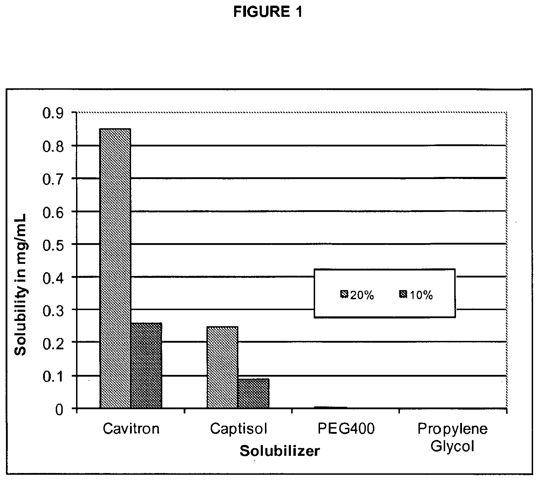

The aqueous solubility of trametinib added in hydroxypropyl B-cyclodextrin (Cavitron), sulfobutylether B-cyclodextrin (Captisol), polyethylene glycol (PEG), and propylene glycol formulation is demonstrated in Figure 1. Cavitron exhibited the greatest solubility followed by captisol. Trametinib was generally not soluble in PEG or propylene glycol solution.

### Concentration of trametinib

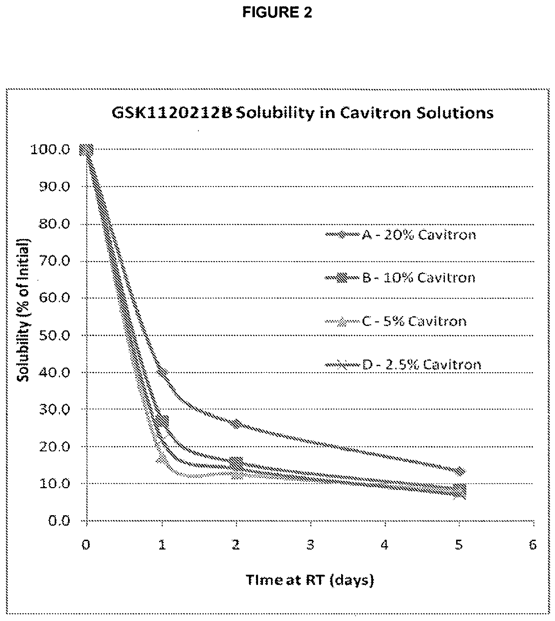

The effect of cavitron concentration and time on trametinib solubility, is demonstrated in Figure 2. In this experiment the initial trametinib concentration was 1 mg/mL and after five days trametinib concentration is reduced to ∼7-10% of the initial concentration. The results in Figure 2 indicate that, under the conditions utilized therein, when the starting concentration of Compound B is ∼ 0.05 mg/mL no significant amount of the drug will precipitate out of solution.

### Addition of HPMC

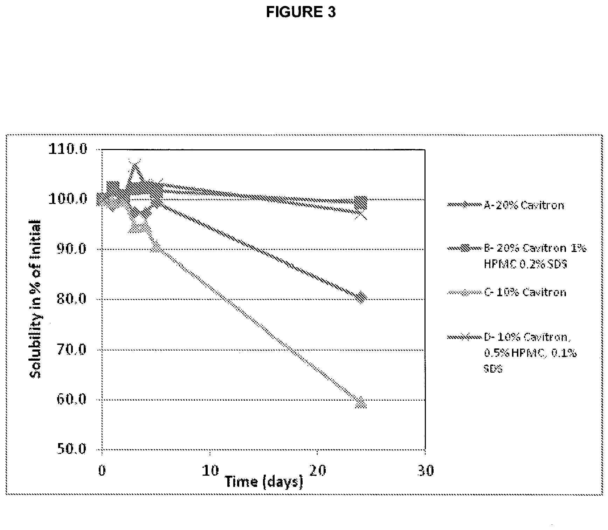

Figure 3 demonstrates that the presence of HPMC in a cavitron solution increases the stability of trametinib solution by inhibiting the precipitation of the trametinib out of the solution.

### Solubility profile of trametinib

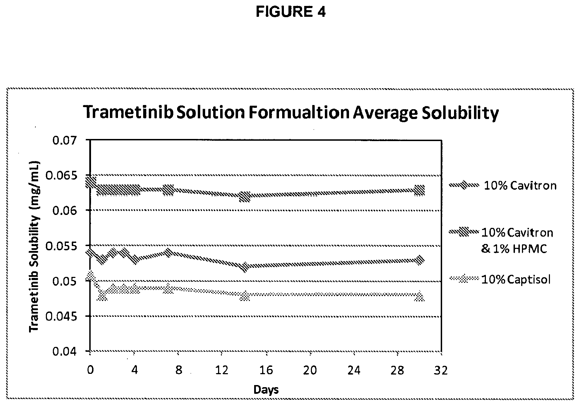

Figure 4 demonstrated the solubility profile of trametinib in cavitron and captisol solutions as a function of time. Both solubilizing agents were able to maintain the solubility and stabilize the solution for 30 days at storage conditions of 25 °C and 60% relative humidity.

Subsequently, antimicrobial effectiveness testing showed that cavitron, in the presence of trametinib, promoted the growth of fungi (mold) and required higher levels of preservatives for microbial stability.

### Flavoring

The taste of trametinib has been summarized as being bitter. The taste perception of a solution formulation was assessed by the Astree® electronic tongue (e-tongue). The e-tongue measures and maps the relative repartition and proximity of the taste between an active suspension formulation and its matching placebo. The e-tongue measurements are analyzed by principal component analysis (PCA). It is assumed that the placebo represents the ideal "target" taste profile, since the bitter active component is not present. Therefore, masking of bitterness or taste proximity is quantified using the euclidean distance between the active and placebo formulations on the PCA map, with a smaller distance indicating a flavor that is doing a better job of masking and therefore bringing the active and placebo e-Tongue "fingerprint" closer together. The discrimination index (DI in %) takes into account the difference between the center of gravity of the sensors output for each pair of formulation as well as the dispersion within the sensors output for the formulations. The higher the value of discrimination index (closer to 100%), the less similarity between the formulations and less masking occurred.

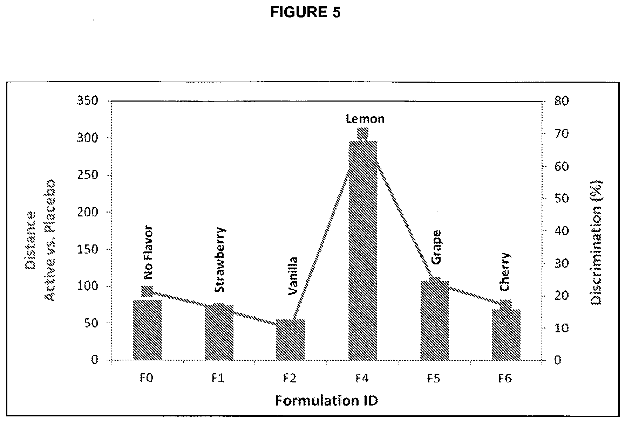

Five different flavors (Strawberry, Vanilla, Lemon, Grape, and Cherry) were tested at 0.3% in trametinib dimethyl sulfoxide solution formulation and its matching placebo to assess their masking efficiency. The results are shown in Figure 5. Not all the flavors decreased the distance of the unmasked trametinib dimethyl sulfoxide solution. Specifically, formulations 4 (Lemon flavored) and 5 (Grape flavored) showed an increase in both distance and discrimination index and therefore are considered less effective in masking the taste of the solution. Formulations 1 (Strawberry), 2 (Vanilla), and 6 (Cherry) showed smaller distance values and discrimination indices compared to the unflavored formulation (F0), indicating an improvement in the taste of the active solution. The discrimination index for formulations F1, F2, and F6 is less than 20% suggesting similarity in the taste masking effectiveness of these three formulations. Based on these results the flavors can be rank in terms of taste masking effectiveness for trametinib dimethyl sulfoxide solution as Vanilla > Cherry > Strawberry. Strawberry flavor has an added advantage because of its high aroma. Human evaluation of the taste was assessed in a relative bioavailability study via a questionnaire and the overall response was that the formulation is acceptable and the taste is not bitter.

In one embodiment, there is provided a direct powder blend formulation comprising of < 1.0 w/w%, preferably less than 0.04% w/w, micronized 5 N-(3-{3-cyclopropyl-5-[(2-fluoro-4-iodophenyl)amino]-6,8-dimethyl-2,4,7-trioxo-3,4,6,7-tetrahydropyrido[4,3-d]pyrimidin-1(2H)-yl}phenyl) acetamide dimethyl sulfoxide, 50.0 to 80.0 w/w% sulfobutylether B-cyclodextrin as a solubilizer, about 4.9 w/w% citric acid and about 4.2 w/w% sodium phosphate as buffers, 5.0 to 15.0 w/w% of sucralose as a sweetener, 0.2 to 2.0 w/w% methylparaben as an antimicrobial preservative, 1.0 to 3.0 w/w% potassium sorbate as an antimicrobial preservative and 1.0 to 5.0 w/w% strawberry flavor.

The components of the direct powder blend may be combined in any order, either individually or with two or more components of the blend being pre-mixed. According to one embodiment, sulfobutylether B-cyclodextrin and sucralose are combined and pre-mixed dry prior to combination with the other ingredients. According to one embodiment, all the excipients are mixed together and then the active pharmaceutical ingredient (API) is placed between the two halves of the pre-blended mix.

In one embodiment, there is provided an oral solution comprising 5 N-(3-{3-cyclopropyl-5-[(2-fluoro-4-iodophenyl)amino]-6,8-dimethyl-2,4,7-trioxo-3,4,6,7-tetrahydropyrido[4,3-d]pyrimidin-1(2H)-yl}phenyl) acetamide dimethyl sulfoxide, sulfobutylether B-cyclodextrin as a solubilizer, citric acid and sodium phosphate as buffers, methylparaben and potassium sorbate as an antimicrobial preservative, sucralose as a sweetener, strawberry flavor, and water.

In one embodiment, there is provided an oral solution comprising 5 N-(3-{3-cyclopropyl-5-[(2-fluoro-4-iodophenyl)amino]-6,8-dimethyl-2,4,7-trioxo-3,4,6,7-tetrahydropyrido[4,3-d]pyrimidin-1(2H)-yl}phenyl) acetamide dimethyl sulfoxide, sulfobutylether B-cyclodextrin as a solubilizer, citric acid and sodium phosphate as buffers, Hypromellose as a solubilizer and surfactant, methylparaben and potassium sorbate as an antimicrobial preservative, sucralose as a sweetener, strawberry flavor, and water.

The invented powder for oral solution (POS) may be administered in therapeutically effective amounts to treat or prevent a disease state, e.g., as described in the above referenced International Application No. PCT/JP2005/011082, and United States Patent Publication No. US 2006/0014768.

A method of this invention of inhibiting MEK activity in humans comprises administering to a subject in need of such activity a therapeutically effective amount of a direct powder blend formulation of the present invention.

The invention also provides for the use of Compound A in the manufacture of a direct powder blend formulation of the present invention.

The invention also provides for the use of Compound A in the manufacture of a direct powder blend formulation of the present invention for use in treating cancer.

The invention also provides for the use of Compound A in the manufacture of a direct powder blend formulation of the present invention for use in inhibiting MEK.

The invention also provides for a direct powder blend formulation for use as a MEK inhibitor which comprises Compound A and a pharmaceutically acceptable carrier of the present invention.

The invention also provides for a direct powder blend formulation for use in the treatment of cancer which comprises Compound A and a pharmaceutically acceptable carrier of the present invention.

The invention also provides for a direct powder blend formulation for use in inhibition MEK which comprises Compound A and a pharmaceutically acceptable carrier of the present invention.

Without further elaboration, it is believed that one skilled in the art can, using the preceding description, utilize the present invention to its fullest extent. Therefore, the following examples are to be construed as merely illustrative and not a limitation of the scope of the present invention.

All the excipients utilized herein are standard pharmaceutical grade excipients available from numerous manufacturers well known to those in the art.

### EXAMPLES

As used herein the symbols and conventions used in these processes, schemes and examples are consistent with those used in the contemporary scientific literature, for example, the Journal of the American Chemical Society or the Journal of Biological Chemistry. Unless otherwise indicated, all temperatures are expressed in °C (degrees Centigrade).

### Example 1

### Formulation preparation

### (i) Trametinib powder blend for reconstitution formulation (Example, batch size 10,000 grams)

A four step secondary manufacturing process was used for the powder for reconstitution to a solution formulation which includes Blending, Milling, Blending and Filling. The first process step was blending, during this phase of the process all excipients were screened through a 20 mesh screen and trametinib was screened through a 35 mesh screen and transfered to a suitable blender such as a Servolift 50 L bin blender. All the materials with exception of the trametinib were blended for 10 minutes at 20 +/- 3 rpm. The second process step was milling, this unit operation was used to delump and narrow the particle size distribution of the blend using a Quadro® Co-mil® assembled with a 032C conidur screen (2A032C02916) at 2000 rpm. In the third step, the delumped material was placed back into the Blender and blended at 20 +/- 3 rpm for 10 minutes. Subsequently, trametinib is added between the two halves of the blended mix and blended for 40 minutes at 20 +/- 3 rpm to achieve uniform distribution of the drug substance and excipients.

The process of Example 1 resulted in a composition having the following composition shown in Table 1.

The target amount 13.126 grams of powder is designed to be reconstituted with 90 mL of vehicle (sterilized or purified water) to achieve a final concentration of Compound B) of 0.05 mg/mL.

**Table 1**

| **INGREDIENTS** | **UNIT FORMULA (g/bottle) (w/w%)** | **FUNCTION** |
|---|---|---|
| TRAMETINIB DIMETHYLSULFOXIDE, MICRONIZED ACTIVE SUBSTANCE | 0.00564 (0.043%) | Active substance |
| Sulfobutylether B-Cyclodextrin (Captisol) | 10.0000 (76.187%) | Solubilizer |
| Citric acid monohydrate | 0.6500 (4.952%) | Buffer |
| Sodium Phosphate, Dibasic, Anhydrous | 0.5500 (4.190%) | Buffer |
| Sucralose powder | 1.4000 (10.666%) | Sweetener |
| Methylparaben | 0.0800 (0.609%) | Preservative |
| Potassium Sorbate | 0.2100 (1.600%) | Preservative |
| Strawberry flavor | 0.2300 (1.752%) | Flavor |
| Total | **13.12600** | |

| **RECONSTITUTION VEHICLE** | | |
|---|---|---|
| Purified Water | 90 mL | Vehicle |

### Example 2

### Formulation preparation

Table 2 depicts a qualitatively similar formulation to Example 1. Both formulations were manufactured using the same unit operations and processing parameters. In Example 2 Captisol is replaced with Cavitron.

The process of Example 2 resulted in a formulation having the composition shown in Table 2.

**Table 2**

| **INGREDIENTS** | **UNIT FORMULA (g/bottle) (w/w%)** | **FUNCTION** |
|---|---|---|
| TRAMETINIB DIMETHYL SULFOXIDE, MICRONIZED ACTIVE SUBSTANCE | 0.00564 (0.043%) | Active substance |
| Hydroxypropyl B-Cyclodextrin (Cavitron) | 10.0000 (76.187%) | Solubilizer |
| Citric acid monohydrate | 0.6500 (4.952%) | Buffer |
| Sodium Phosphate, Dibasic, Anhydrous | 0.5500 (4.190%) | Buffer |
| Sucralose powder | 1.4000 (10.666%) | Sweetener |
| Methylparaben | 0.0800 (0.609%) | Preservative |
| Potassium Sorbate | 0.2100 (1.600%) | Preservative |
| Strawberry flavor | 0.2300 (1.752%) | Flavor |
| Total | **13.12600** | |

| **RECONSTITUTION VEHICLE** | | |
|---|---|---|
| Purified Water | 90 mL | Vehicle |

### Example 3

### Formulation preparation

Table 3 depicts a qualitatively similar formulation to Example 1. Both formulations were manufactured using the same unit operations and processing parameters. In Example 3 Captisol is replaced with Cavitron and Hypromellose (HPMC).

The process of Example 3 resulted in a formulation having the composition shown in Table 3.

**Table 3**

| **INGREDIENTS** | **UNIT FORMULA (g/bottle) (w/w%)** | **FUNCTION** |
|---|---|---|
| TRAMETINIB DIMETHYL SULFOXIDE, MICRONIZED ACTIVE SUBSTANCE | 0.00564 (0.043%) | Active substance |
| Hydroxypropyl B-Cyclodextrin (Cavitron) | 10.0000 (75.440%) | Solubilizer |
| Citric acid monohydrate | 0.6500 (4.904%) | Buffer |
| Sodium Phosphate, Dibasic, Anhydrous | 0.5500 (4.149%) | Buffer |
| Sucralose powder | 1.4000 (10.562%) | Sweetener |
| Hypromellose | 0.1400 (1.056%) | Solubilizer and Surfactant |
| Methylparaben | 0.0800 (0.604%) | Preservative |
| Potassium Sorbate | 0.2000 (1.509%) | Preservative |
| Strawberry flavor | 0.2300 (1.735%) | Flavor |
| Total | **13.256** | |

| **RECONSTITUTION VEHICLE** | | |
|---|---|---|
| Purified Water | 90 mL | Vehicle |

### Example 4

### Formulation preparation

Table 4 depicts a qualitatively similar formulation to Example 1. Both formulations were manufactured using the same unit operations and processing parameters. In Example 4 no preservative is used and the Captisol is replaced with 5g of Cavitron.

The process of Example 4 resulted in a formulation having the composition shown in Table 4.

**Table 4**

| **INGREDIENTS** | **UNIT FORMULA (g/bottle) (w/w%)** | **FUNCTION** |
|---|---|---|
| TRAMETINIB DIMETHYL SULFOXIDE, MICRONIZED ACTIVE SUBSTANCE | 0.00564 (0.07125%) | Active substance |
| Sulfobutylether B-Cyclodextrin (Captisol) | 5.0000 (63.16608%) | Solubilizer |
| Citric acid monohydrate | 0.6500 (8.21159%) | Buffer |
| Sodium Phosphate, Dibasic, Anhydrous | 0.5500 (6.94826%) | Buffer |
| Sucralose powder | 1.4000 (17.6865%) | Sweetener |
| Methylparaben | 0.0800 (1.01065%) | Preservative |
| Strawberry flavor | 0.2300 (2.90563%) | Flavor |
| Total | **7.91564** | |

| **RECONSTITUTION VEHICLE** | | |
|---|---|---|
| Purified Water | 90 mL | Vehicle |

While the preferred embodiments of the invention are illustrated by the above, it is to be understood that the invention is not limited to the precise instructions herein disclosed and that the right to all modifications coming within the scope of the following claims is reserved.

## Claims

1. A direct powder blend formulation comprising:
a) an amount of a drug, which is N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide solvate, and a solubilizer;
wherein,
b) the solubilizer is selected from: Hydroxypropyl B-Cyclodextrin; Sulfobutylether B-Cyclodextrin; a combination of Hydroxypropyl B-Cyclodextrin and Sulfobutylether B-Cyclodextrin; a combination of Hydroxypropyl B-Cyclodextrin and Hypromellose; a combination of Sulfobutylether B-Cyclodextrin and Hypromellose; and Hypromellose.

2. The direct powder blend formulation of claim 1, wherein the solubilizer is Sulfobutylether B-Cyclodextrin.

3. The direct powder blend formulation of claim 1, wherein the soubilizer is present in an amount of greater than about 60% (by weight).

4. A direct powder blend formulation comprising:
a) an amount of a drug, which is N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide solvate, a solubilizer, buffer and sweetener:
wherein,
b) the solubilizer is selected from: Hydroxypropyl B-Cyclodextrin; Sulfobutylether B-Cyclodextrin; a combination of Hydroxypropyl B-Cyclodextrin and Sulfobutylether B-Cyclodextrin; a combination of Hydroxypropyl B-Cyclodextrin and Hypromellose; a combination of Sulfobutylether B-Cyclodextrin and Hypromellose; and Hypromellose; and
c) the particle size distribution of the drug is at least 90% of the particles are from 1 to 20 microns.

5. A direct powder blend formulation comprising:
a) an amount of a drug, which is N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide solvate, a solubilizer, buffer and sweetener:
wherein,
b) the solubilizer is selected from: Hydroxypropyl B-Cyclodextrin; Sulfobutylether B-Cyclodextrin; a combination of Hydroxypropyl B-Cyclodextrin and Sulfobutylether B-Cyclodextrin; and Hypromellose; and
c) the particle size distribution of the drug is at least 90% of the particles are from 1 to 20 microns.

6. An oral solution comprising:
a) an amount of a drug, which is N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide solvate, a solubilizer, preservative, buffer, sweetener, and an aqueous vehicle:
wherein,
b) the solubilizer is selected from: Hydroxypropyl B-Cyclodextrin; Sulfobutylether B-Cyclodextrin; a combination of Hydroxypropyl B-Cyclodextrin and Sulfobutylether B-Cyclodextrin; a combination of Hydroxypropyl B-Cyclodextrin and Hypromellose; a combination of Sulfobutylether B-Cyclodextrin and Hypromellose; or Hypromellose.

7. An oral solution comprising:
a) an amount of a drug, which is N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide solvate, a solubilizer, and an aqueous vehicle:
wherein,
b) the solubilizer is selected from: Hydroxypropyl B-Cyclodextrin; Sulfobutylether B-Cyclodextrin; a combination of Hydroxypropyl B-Cyclodextrin and Sulfobutylether B-Cyclodextrin; a combination of Hydroxypropyl B-Cyclodextrin and Hypromellose; a combination of Sulfobutylether B-Cyclodextrin and Hypromellose; or Hypromellose.

8. An oral solution comprising:
a) an amount of a drug, which is N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide solvate, a solubilizer, preservative, buffer, sweetener, flavor, and an aqueous vehicle:
wherein,
b) the solubilizer is selected from: Hydroxypropyl B-Cyclodextrin; Sulfobutylether B-Cyclodextrin; a combination of Hydroxypropyl B-Cyclodextrin and Sulfobutylether B-Cyclodextrin; a combination of Hydroxypropyl B-Cyclodextrin and Hypromellose; a combination of Sulfobutylether B-Cyclodextrin and Hypromellose; or Hypromellose.

9. An oral solution comprising:
a) an amount of a drug, which is N-{3-[3-cyclopropyl-5-(2-fluoro-4-iodo-phenylamino)-6,8-dimethyl-2,4,7-trioxo-3,4,6,7-tetrahydro-2H-pyrido[4,3-d]pyrimidin-1-yl]phenyl}acetamide dimethyl sulfoxide solvate, a solubilizer, preservative, buffer, sweetener, surfactant, flavor, and an aqueous vehicle:
wherein,
b) the solubilizer is selected from: Hydroxypropyl B-Cyclodextrin; Sulfobutylether B-Cyclodextrin; a combination of Hydroxypropyl B-Cyclodextrin and Sulfobutylether B-Cyclodextrin; a combination of Hydroxypropyl B-Cyclodextrin and Hypromellose; a combination of Sulfobutylether B-Cyclodextrin and Hypromellose; or Hypromellose.

10. A formulation according to any one of claims 1 to 5 for use in the treatment of cancer in a human.

11. A formulation according to any one of claims 1 to 5 for use in inhibiting MEK in a human.

12. A solution according to any one of claims 6 to 9 for use in the treatment of cancer in a human.

13. A solution according to any one of claims 6 to 9 for use in inhibiting MEK in a human.
